# EUROPEAN PATENT APPLICATION

(11) **EP 4 032 490 A1**
(43) Date of publication of application: **27.07.2022**
(21) Application number: 22152879.7
(22) Date of filing: 24.01.2022
(51) Int. Cl.: A61B 17/22, A61B 17/221

(54) **ANCHORING AND NON-OCCLUDING INTRAVASCULAR DEVICE FOR USE DURING CLOT REMOVAL VIA ASPIRATION AND/OR MECHANICAL EXTRACTION DEVICE**

(30) Priority: 25.01.2021 US 202117157878
(71) Applicant: DePuy Synthes Products, Inc., Raynham, MA 02767 (US)
(72) Inventor: GOROCHOW, Lacey, RAYNHAM, 02767 (US); MAGEN, Eytan, RAYNHAM, 02767 (US)
(74) Representative: Carpmaels & Ransford LLP

(57) **Abstract**

An anchoring and non-occluding intravascular device including a guide catheter and an anchor supporting structure disposed on the guide catheter and deployable from a contracted state to an enlarged state having an enlarged outer diameter relative to that while in a contracted state. In both the contracted and in the enlarged state the anchor supporting structure is configured to allow passage of blood distally beyond the anchor supporting structure; and the anchoring is provided exclusively by the anchor supporting structure. In use, prior to capture and retrieval of a clot within a vessel, the anchor supporting structure being deployed from the contracted state to the enlarged state. While in the enlarged state, the deployed anchor supporting structure anchoring in place the guide catheter in the vessel while still allowing blood flow through the vessel distally beyond the deployed anchor supporting structure.

## Description

### BACKGROUND OF THE INVENTION

### Field of the Invention

The present invention relates to an anchoring and non-occluding intravascular device for use during removal of clots via aspiration and/or mechanical extraction device. In particular, the present invention is directed to an intravascular device for use during clot removal that prohibits or minimizes movement (i.e., anchors) of a guide catheter positioned within the artery/vessel without occluding blood flow.

### Description of Related Art

In the area of interventional neurovascular treatments, conventional balloon guide catheters are widely used in a variety of intravascular medical procedures including thrombectomy procedures. Using standard imaging techniques (e.g., X-ray radiology), a guidewire is advanced through a vessel across and beyond a target clot, thrombus, occlusion or blockage followed thereafter by a guide catheter tracked over the guidewire. The guidewire is withdrawn and a mechanical thrombectomy device (e.g., stent retriever or stentriever) is advanced through the lumen of the guide catheter until proximate the distal end of the guide catheter. In this position, at least a distal portion of the mechanical thrombectomy device extends beyond the target clot, occlusion or blockage. The guide catheter is then partially withdrawn unsheathing (i.e., freeing) the entire mechanical thrombectomy device to deploy across the occluding blockage. No longer constrained by the guide catheter, the mechanical thrombectomy device automatically self-expands to an enlarged maximum diameter. As it self-expands the mechanical thrombectomy device engages the thrombus therein, applying a radial force that compresses the thrombus against the vessel wall immediately restoring partial reperfusion of the distal vasculature. After passage of a predetermined period of time (e.g., typically approximately 2-5 minutes) the thrombus is sufficiently embedded in the spaces or openings between struts of the expanded mechanical thrombectomy device. At this late stage of the procedure, after capture but subsequent to removal of the captured clot, a balloon mounted proximate the distal end of the guide catheter may be inflated to provide back-up support to the guide catheter during subsequent retrieval of the clot and/or aspiration. With the balloon inflated, the guide catheter and mechanical thrombectomy device with the clot embedded therein are simultaneously withdrawn as a single unit. Since the balloon is not inflated until late in the process (following deployment of the mechanical thrombectomy device resulting in capture/engagement/embedding in the thrombus), prior to inflation of the balloon no back-up support is provided to the guide catheter to ensure that it is maintained in position at the critical moment during the medical procedure of capturing and retrieving the clot. During such critical time when the clot is being retrieved, it is imperative that the interventionalist's focus not be diverted resulting in possible unwanted movement of the guide catheter. Heretofore, back-up support to prevent or minimize movement of the guide catheter during capture and retrieval of the clot has been limited to modifying the construction design of the guide catheter itself. That is, making the construction of the guide catheter more complex at the expense of increasing manufacturing cost; utilizing a stiffer build construction causing potential damage to vessels during tracking; and limiting the degree of lubricity for tracking the balloon guide catheter making advancement through the vessel more difficult.

In conventional balloon guide catheters, inflation of the balloon provides back-up support (i.e., prevents or minimizes movement in a proximal direction) for the guide catheter during retrieval of the microcatheter and stentriever with the clot already engaged therein. Anchoring of the guide catheter is achieved when the inflated balloon having a spherical or elliptical shape physically contacts the inner wall of the vessel thereby maintaining in position the guide catheter. Prevention or minimization of movement of such conventional guide catheter using the balloon also disadvantageously occludes (cuts off completely) blood flow through the artery in a distal direction beyond the deployed balloon. Maintaining blood flow in the artery during mechanical thrombectomy procedures is time dependent for safety. Occluding blood flow may result in potential serious harm to the patient. Accordingly, use of the inflated balloon as an anchoring device is limited as sparingly as possible. The balloon is inflated or expanded for the briefest duration only during specific procedures in which the guide catheter is most susceptible to movement. Typically, these specific procedures are during retraction in a proximal direction of the microcatheter along with the mechanical thrombectomy device and/or aspiration of the clot, blockage or occlusion.

It is therefore desirable to develop an improved intravascular device that provides back-up support (anchoring) of the guide catheter within the artery/vessel prior to capture of the clot and throughout the procedure non-occluding (i.e., neither cutting off completely nor entirely stopping) blood flow through the artery/vessel.

### Summary of the Invention

An aspect of the present invention relates to an improved intravascular device that provides back-up support for the guide catheter during capture and retrieval of the clot without cessation or occlusion of blood flow through the artery.

Another aspect of the present invention is directed to an anchoring and non-occluding intravascular device having a guide catheter including a catheter shaft with a proximal end, an opposite distal end, and a lumen defined longitudinally therein. The intravascular device also including an anchor supporting structure disposed on the guide catheter and deployable from a contracted state to an enlarged state having an enlarged outer diameter relative to that while in a contracted state. In both the contracted and in the enlarged state the anchor supporting structure is configured to allow passage of blood distally beyond the anchor supporting structure; and the anchoring is provided exclusively by the anchor supporting structure.

Still another aspect of the present invention is directed to a method for using an anchoring and non-occluding intravascular device as described in the preceding paragraph, wherein the method includes the step of prior to capture and retrieval of a clot within a vessel, deploying the anchor supporting structure from the contracted state to the enlarged state. While in the enlarged state, the deployed anchor supporting structure anchoring in place the guide catheter in the vessel while still allowing blood flow through the vessel distally beyond the deployed anchor supporting structure. The anchoring of the guide catheter within the vessel being provided exclusively by the anchor supporting structure.

### Brief Description of the Drawing

The foregoing and other features of the present invention will be more readily apparent from the following detailed description and drawings illustrative of the invention wherein like reference numbers refer to similar elements throughout the several views and in which:

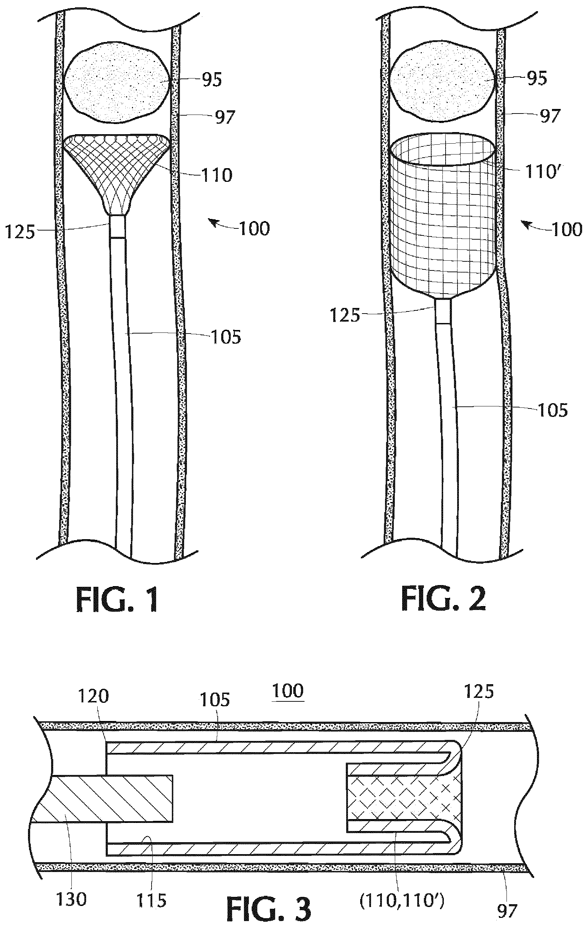

Figure 1 is a view within the artery/vessel an exemplary anchoring and non-occluding intravascular device in accordance with the present invention including a funnel or cone shape mesh anchor supporting structure illustrated in a deployed state (expanded state) mounted to a distal end of a guide catheter;
Figure 2 is a view within the artery vessel of another exemplary anchoring and non-occluding intravascular device in accordance with the present invention including a cylindrical shape mesh anchor supporting structure terminating in a funnel shape proximal end illustrated in a deployed state (expanded state) mounted to a distal end of a guide catheter;
Figure 3 is a cross-sectional longitudinal view within the artery/vessel of the anchoring and non-occluding intravascular device of Figure 1 while the mesh anchor supporting structure in a contracted/compressed state is tucked/flipped/inverted within itself and received in the interior of the lumen at the distal end of the guide catheter;

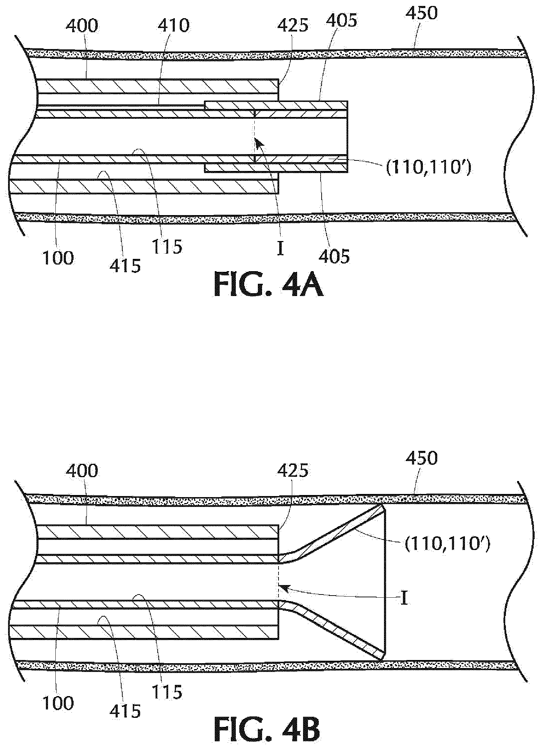

Figure 4A is a partial cross-sectional longitudinal view within the artery/vessel of another configuration of the present inventive anchoring and non-occluding intravascular device with the mesh anchor supporting structure maintained in a compressed state by a protective sleeve;
Figure 4B is a partial cross-sectional longitudinal view within the artery/vessel of the anchoring and non-occluding intravascular device of Figure 4A with the mesh anchor supporting structure in an expanded/enlarged state (deployed) following withdraw in a proximal direction of the protective sleeve;

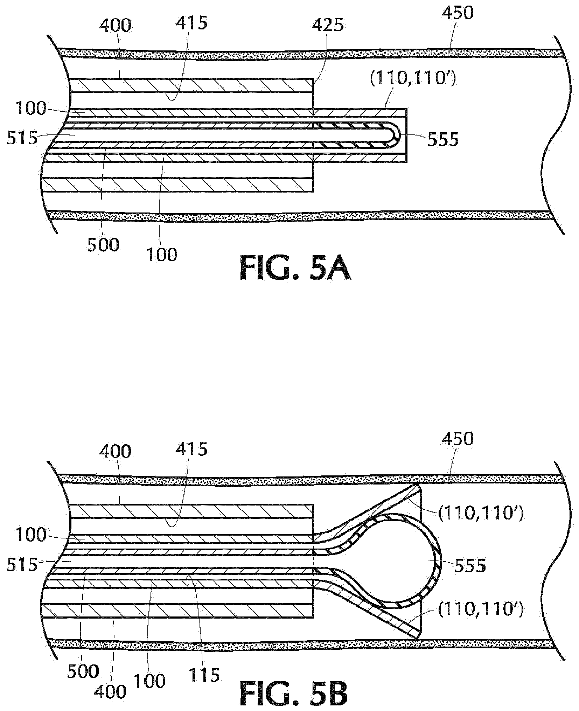

Figure 5A is a partial cross-sectional longitudinal view in the artery/vessel of yet another configuration of the present inventive anchoring and non-occluding intravascular device with a mesh anchor supporting structure deployable (enlarged/expanded) by a radially outward force imposed by an inflatable compliant balloon, wherein the compliant balloon is illustrated in a deflated state and the mesh anchor supporting structure is in a compressed state;
Figure 5B is a partial cross-sectional longitudinal view in the artery/vessel of the anchoring and non-occluding intravascular device of Figure 5A, wherein the balloon is illustrated in an inflated state and the mesh anchor supporting structure is in an expanded/enlarged state;

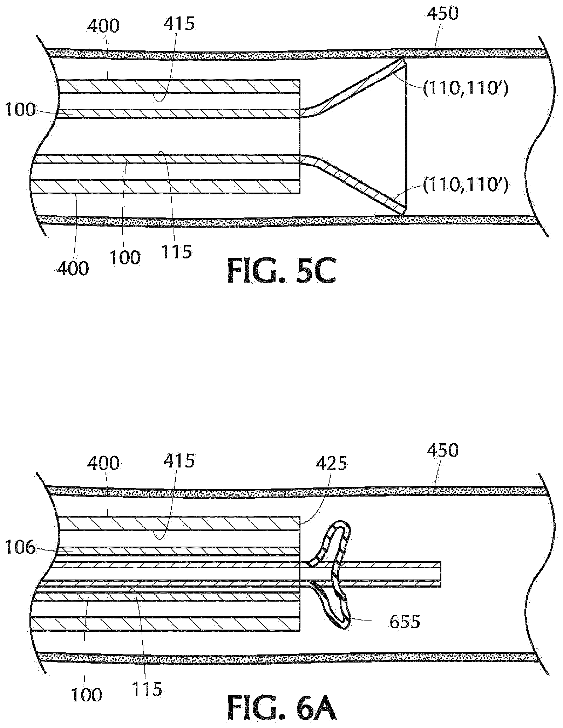

Figure 5C is a partial cross-sectional longitudinal view in the artery/vessel of the anchoring and non-occluding intravascular device of Figure 5A illustrating the mesh anchor supporting structure maintained in an expanded/enlarged state and in place within the vessel following withdraw of the deflated balloon;
Figure 6A is a partial cross-sectional longitudinal view in the artery/vessel of still another configuration of the present inventive anchoring and non-occluding intravascular device with a deflated C-shaped balloon anchor supporting structure disposed about the outer perimeter of a guide catheter;

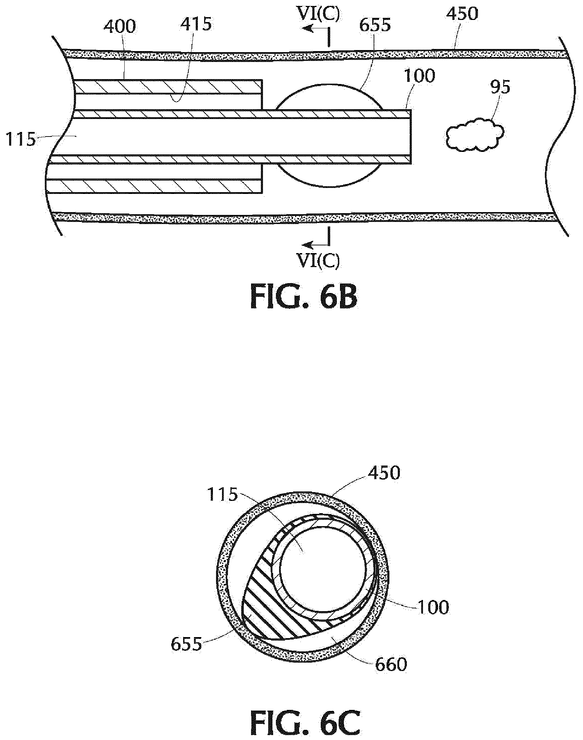

Figure 6B is a partial cross-sectional longitudinal view in the artery/vessel of the anchoring and non-occluding intravascular device of Figure 6A disposed on a proximal side of a target clot, wherein the C-shaped balloon anchor supporting structure is depicted in an inflated state partially around the circumference of the catheter shaft of the guide catheter;
Figure 6C is a radial cross-sectional view along lines VI(C) - VI(C) in Figure 6B depicting the spaces defined between the inner wall of the artery/vessel and the inflated C-shaped balloon anchor supporting structure anchoring the guide catheter in position within the artery/vessel yet allowing the passage of blood distally beyond the inflated balloon.

### Detailed Description of the Invention

The terms "distal" or "proximal" are used in the following description with respect to a position or direction relative to the treating physician or medical interventionalist. "Distal" or "distally" are a position distant from or in a direction away from the physician or interventionalist. "Proximal" or "proximally" or "proximate" are a position near or in a direction toward the physician or medical interventionalist. The terms "occlusion", "clot" or "blockage" are used interchangeably.

The present inventive anchoring and non-occluding intravascular device provides back-up support that prevents or minimizes proximal movement of the guide catheter during subsequent medical procedures (e.g., aspiration and/or mechanical retrieval of the clot) without occluding (e.g., cutting off completely or entirely stopping) blood flow through the artery/vessel distally beyond the deployed anchoring and non-occluding intravascular device. In some configurations the present inventive anchoring and non-occluding intravascular device includes a self-expanding anchor structure (e.g., skeleton, frame, perforated or mesh structure) having a plurality of openings defined therein that allows blood flow distally beyond when deployed. While in an expanded/enlarged/deployed state, the anchor structure maintains the position of the guide catheter within the artery/vessel during capture and retrieval of the occlusion while permitting blow flow distally beyond the deployed anchor structure. In another embodiment of the present invention, rather than using a self-expanding structure, an inflatable balloon or other radially outward mechanical expanding device may be used with the singular or only purpose to deploy (expand/enlarge) the anchor supporting structure (i.e., the balloon in this configuration is not being used as the anchor supporting structure itself to maintain the guide catheter in place within the artery/vessel). Yet in still another embodiment of the present invention, an inflatable balloon itself may serve as the anchor supporting structure to maintain the guide catheter in position (prevent or minimize back-up) within the artery/vessel, but such design of the inflatable balloon still intentionally allows at blood flow (i.e., the inflated balloon neither occludes nor stops blood flow) distally beyond the deployed balloon.

Referring to Figures 1 & 2, one configuration of anchoring and non-occluding intravascular devices 100 in accordance with the present invention includes an expandable frame, mesh, skeleton or cage (110, 110') as an anchor supporting structure mounted directly to the catheter shaft 105. The anchor supporting structure (110, 110') has a plurality of spaces or openings defined therein that allow blood flow therethrough when deployed in the artery or vessel. Preferably, the anchor supporting structure is made of Nitinol or other shape memory alloy that is biocompatible. Due to the material and design, the Nitinol anchor supporting structure is compressible to a reduced diameter (contracted/compressed/reduced diameter state), but when deployed automatically self-expands to its original enlarged diameter (expanded/enlarged diameter state) upon withdrawal of an external mechanical force retaining the device in its contracted/compressed state (e.g., provided by the inner wall of the lumen of the guide catheter through which the anchor supporting structure is delivered to a target site in the artery/vessel). The anchor supporting structure is mounted proximate the distal end 125 of the guide catheter 100, that is, either directly to the terminating distal end/tip of the guide catheter or proximally of the terminating distal end/tip. In the exemplary configurations depicted in Figures 1 & 2, the anchoring support structure (110, 110') is mounted to directly to the terminating distal end/tip 125 of the catheter shaft 105 of the guide catheter 100. In the exemplary embodiment illustrated in Figure 1, the anchoring support structure 110 (when deployed in an expanded/enlarged state) is in the shape of a cone or funnel arranged so that a largest diameter is found at its distal end and tapers radially inwards towards its opposite proximal end having a smallest diameter mounted to the distal end 125 of the shaft 105 of the guide catheter 100. An alternative configuration of the anchoring support structure 110', depicted in Figure 2 in an expanded/enlarged state, is a composite of the cone or funnel shape (illustrated in Figure 1) at its proximal end where mounted to the catheter shaft and at its largest diameter distal end extends in a distal direction into a cylinder of uniform diameter.

In preparation of being introduced into the vessel or artery 97, the anchor supporting structure 110, 110' while maintained in a wrapped-down, folded or otherwise compressed/contracted state (having a reduced outer diameter relative to that while in an expanded/enlarged state) is tucked/flipped/inverted within itself to be received inside the lumen 115 proximate the distal end 125 of the catheter shaft 105 of the guide catheter 100, as illustrated in Figure 3. The anchor supporting structure 110, 110' is maintained in this compressed/contracted state by the inner wall of the lumen 115 of the catheter shaft 105. Together the guide catheter with the anchor supporting structure (while in a compressed/contracted state) tucked/flipped/inverted within itself into the lumen of the catheter shaft are tracked over the guide wire to a position within the artery/vessel on a proximal side of the targeted clot 95. Once positioned at the target site on the proximal side of the clot 95, the anchor supporting structure 110, 110' is deployed by being pushed out from the distal end 125 of the catheter shaft 105 of the guide catheter using a guidewire 130 or some other mechanical pushing device advanced in a distal direction through the lumen 115 of the guide catheter. Upon exiting from the distal end 125 of the catheter shaft 105 the anchor supporting structure 110, 110' automatically self-expands (deploys) to an expanded state (having an enlarged outer diameter in physical contact against the inner wall of the artery/vessel 97) thereby maintaining the position of the guide catheter at the target site within the artery/vessel during subsequent capture and retrieval of the clot, as shown in Figures 1 & 2.

Other mechanical configurations are contemplated for maintaining the anchor supporting structure in a compressed/contracted state having a reduced outer diameter prior to being deployed (during delivery to a target site within the artery/vessel). For instance, the anchor supporting structure may be retained in a compressed/contracted state (having a reduced outer diameter) by a protective sleeve disposed about the outer perimeter of the mesh anchor supporting structure, rather than by the inner wall of the lumen 115 of the catheter shaft 105 of the guide catheter 100 (as depicted in Figures 1-3). Referring to the partial cross-sectional view in Figure 4A, the anchor supporting structure 110, 110' is mounted proximate to or directly to the tip of the distal end of the guide catheter 100. In preparation of the guide catheter 100 being introduced into the proximal end of an ancillary or supplemental catheter 400 to be delivered to a target site in the artery/vessel, a protective sleeve 405 is slid over or wrapped about the outer perimeter of the anchor supporting structure 110, 110' (while wrapped-down to a compressed/contracted state having a reduced diameter) and the distal portion of the guide catheter 100. Protective sleeve 405 maintains the anchor supporting structure 110, 110' in a compressed/contracted state having a reduced outer diameter until removed/withdrawn. As illustrated in Figure 4A, protective sleeve 405 preferably extends from proximate the distal end of the mesh anchor supporting structure 110, 110' in a proximal direction across the interface ("I") with the distal end of the guide catheter 100. The protective sleeve 405 has an outer diameter sized to be receivable within the lumen 415 defined in the ancillary or supplemental catheter 400. Guide catheter 100, protective sleeve 405, and anchor supporting structure 110, 110' may be introduced as a single unit or package into the proximal end of the ancillary catheter 400 and advanced in a distal direction through the lumen 415. After exiting from the distal end 425 of the ancillary catheter 400 positioned on a proximal side of the the clot, blockage or occlusion, the protective sleeve 405 is removed/withdrawn in a proximal direction. Specifically, a suture or wire 410 connected at one end to the protective sleeve 405 and extending proximally the length of the intravascular device to the hub is manipulated (i.e., pulled - withdrawn in a proximal direction), until the anchor supporting structure 110, 110' is free from (i.e., no longer constrained by) the protective sleeve 405 anchoring in place the guide catheter 100 within the artery/vessel 450. No longer constrained by the protective sleeve 405, as illustrated in Figure 4B, the anchor supporting structure 110, 110' automatically transitioning to an expanded state of enlarged outer diameter in physical contact against the inner wall of the artery/vessel 450 thereby preventing or minimizing movement of the guide catheter 100 in the artery/vessel during subsequent capture and retrieval of the clot. Guide catheter 100 being mounted thereto moves simultaneously with the anchor supporting structure 110, 110' during transitioning to the expanded state. For illustrative purposes only the anchor supporting structure 110, 110' is depicted as a funnel or cone shape (similar to that of Figure 1) but alternative shapes are contemplated such as, but not limited to, a cylinder shape or combination cone & cylinder shape (as shown in Figure 2).

As an alternative to employing a self-expanding anchor supporting structure automatically transitioning to an expanded state having an enlarged diameter upon removal of a mechanical constraining device (e.g., lumen of the guide catheter or protective sleeve), an anchor supporting structure may be employed that deploys (i.e., expands in diameter) only upon application of a mechanical radially outward expanding force. In this configuration, an inflatable compliant balloon or other mechanical expansion device may be used, wherein the balloon is used for the sole purpose of deploying the anchor supporting structure to its expanded state having an enlarged outer diameter. In other words, the compliant balloon itself when inflated never physically contacts the inner wall of the artery/vessel and thus no back-up support is provided by the inflated compliant balloon itself. Instead of relying on the balloon, movement (back-up) of the guide catheter is prevented or minimized only by the deployed anchor supporting structure 110, 110' in an expanded state physically contacting the inner wall of the artery/vessel. A series of catheters may be employed in accordance with this embodiment as illustrated in Figures 5A-5C. Disposed within the lumen 415 of the ancillary catheter 400 is the guide catheter 100. Once again, the anchor supporting structure 110, 110' is mounted proximate the distal end (either directly to the distal end of the guide catheter or proximally inward of the distal end) of the guide catheter 100. Advanced in a distal direction within a lumen 105 of the guide catheter 100 is a balloon catheter 500 having an inflation/deflation lumen 505 extending axially in the catheter shaft for inflating/deflating a compliant balloon 555 mounted to the distal end of the catheter shaft. Balloon 555 is aligned in a longitudinal direction within the anchor supporting structure 110, 110'. As with the previous configurations discussed above, initially the ancillary catheter 400 is advanced over a guidewire proximate the clot, blockage or occlusion. Then, the guide catheter 100 with the anchor supporting structure 110, 110' mounted thereto while in a compressed/retracted state (having a reduced diameter) together with the balloon catheter 500 (while the balloon 555 is in a deflated state) are introduced through the lumen 415 of the ancillary catheter 400. The guide catheter 100, compressed anchor supporting structure 110, 110', and balloon catheter 500 with deflated balloon 555 may be introduced all together as a single integrated unit or package. Alternatively, the guide catheter 100 and compressed anchor supporting structure 110, 110' may be introduced first as a unit, followed thereafter by the balloon catheter 500 with deflated balloon 555 together as a subsequent unit. In either case, once the anchor supporting structure 110, 110' exits from the distal end 425 of the ancillary catheter 400, inflation media (e.g., 50% contrast saline solution) is introduced through the inflation/exhaust lumen 515 of the balloon catheter 500 to inflate the balloon 555. Balloon catheter 500 is depicted in Figure 5A-5C as having a single lumen for both inflation/deflation of the balloon 555. Alternatively, balloon catheter 500 may be designed to have separate lumen, that is, one for inflation and a separate lumen for deflation of the balloon 555 arranged coaxially and in an axial direction parallel to one another. As the balloon 555 inflates (expands) radially outward pressure is exerted on the anchor supporting structure 110, 110' causing it to expand (enlarge) in diameter until the free terminating distal end of the anchor supporting structure 110, 110' physically contacts the inner walls of the artery/vessel 450 anchoring it in position, as shown in Figure 5B. After the anchor supporting structure 110, 110' has been deployed (expanded/enlarged), balloon 555 is deflated (by removal or purging of the inflation media via the lumen 515) and withdrawn in a proximal direction together with the balloon catheter 500, leaving in place the guide catheter 100 together with the deployed (expanded) anchor supporting structure 110, 110'. Since the anchor supporting structure 110, 110' in the configuration shown in Figures 5A-5C is not self-expanding, it remains in an expanded state within the artery/vessel following deflation and withdrawal of the balloon 555.

In yet another configuration an inflatable compliant balloon may be used itself as the anchor supporting structure. Contrary to conventional balloon guide catheters wherein the inflated balloon is intended to occlude blood flow, the design of the balloon itself as the anchor supporting structure in this configuration of the present inventive anchoring and non-occluding intravascular device intentionally allows at least a certain amount of blood flow distally beyond the inflated balloon in spaces defined between the inner wall of the artery/vessel and the inflated balloon. To achieve this desired goal, the present inventive balloon anchor supporting structure is geometrically designed so that in an inflated/expanded state the inflated balloon maintains the guide catheter in place within the artery/vessel prior to capture and thereafter retrieval of the clot while permitting blood flow distally beyond the inflated balloon. An ancillary or supplemental catheter 400 tracks over a guide wire within the artery/vessel 450 to a position on a proximal side or face of the clot, blockage or occlusion. A compliant balloon 655 (while in a deflated state) is wrapped partially about the outer perimeter of a guide catheter proximate its distal end. The guide catheter with the wrapped about deflated balloon 655 together are introduced into a proximal end of the lumen 415 of the ancillary or supplemental catheter 400. Figure 6A is a partial cross-sectional longitudinal view of the present inventive anchoring and non-occluding intravascular device in an artery/vessel 450 with the deflated balloon 655 exiting from the distal end 425 of the ancillary and supplemental catheter 400. After exiting from the distal end 425 of the ancillary or supplemental catheter 400 at a target site on a proximal side of the clot, balloon 655 is inflated by introducing an inflation media (e.g., 50% contrast saline solution) delivered through an inflation lumen 106 (depicted in Figure 6A as parallel to the central lumen 115. The inflated balloon 655, as shown in Figure 6B, partially surrounds the outer perimeter of the guide catheter 100. Figure 6C is a radial cross-sectional view along lines VI(C) - VI(C) of Figure 6B illustrating the C-shape or U-shape profile of the inflated balloon in a radial plane perpendicular to the longitudinal axis through the guide catheter 100. The inflated balloon 655 (whether C-shaped or U-shaped) comprises two arm sections each terminating in a respective free end and an intermediate curved section disposed between the two arm sections. As shown in the radial cross-sectional view of Figure 6C, the outer surface of at least a portion of the intermediate curved section of the inflated balloon 655 physically contacts the inner wall of the vessel/artery 450 thereby anchoring the guide catheter 100 in position within the vessel/artery providing back-up support during capture and retrieval of the clot. The two arm sections comprising the inflated balloon 655, however, do not physically contact the inner wall of the vessel/artery 450 thereby defining a space 660 that allows blood flow therethrough distally beyond the inflated balloon 655.

In operation, a guidewire is introduced into the vessel/artery to a desired location on a proximal side of the target clot, occlusion or blockage. Typically, the guidewire is introduced into the body via the carotid artery for anterior vasculature access or the subclavian artery for posterior vascular access. However, other arteries/vessels may be selected based on the location in the body of the targeted clot, blockage or occlusion. Once the guidewire is properly positioned, an ancillary or supplemental catheter is advanced over the guidewire (i.e., the guidewire is received within the lumen of the ancillary or supplemental catheter) to the proximal side of the clot, blockage or occlusion using standard imaging, e.g., X-ray imaging. Next, the guide catheter 100 together with the anchor supporting structure (in a retracted or compressed state) is introduced into the proximal end of the ancillary or supplemental catheter 400 and advanced through the lumen to the opposite distal end. Upon emerging from the distal end of the ancillary or supplemental catheter, the anchor supporting structure 110, 110' is deployed to an expanded state. Deployment of the anchor supporting structure depends on the specific design of the device. In Figures 1-3, the anchor supporting structure 110, 110' is self-expanding and deploys automatically upon exiting/emerging from the distal end of the ancillary or supplemental catheter. Whereas, the self-expanding anchor supporting structure 110, 110' of Figures 4A & 4B, automatically deploys upon removal of the protective sleeve 405 withdrawn in a proximal direction. In Figures 5A-5C, rather than be self-expanding, the anchor supporting structure 110, 110' deploys only upon application of a radially outward expansion force (e.g., imparted by balloon 555 when inflated (enlarged)). Whereas in the case of the C-shaped or U-shaped balloon anchor supporting structure of Figures 6A-6C the balloon 655 itself is the anchor supporting structure that is enlarged (deployed) by inflating with an inflation fluid. Regardless of the design, in an enlarged state (deployed) the anchor supporting structure in accordance with the present invention provides back-up support to the distal end of the guide catheter during clot capture and retrieval using a mechanical device and/or aspiration while still allowing at least some blood flow distally beyond the deployed anchor supporting structure. Once the anchor supporting structure has been deployed, thereafter a microcatheter can be advanced through the lumen of the guide catheter to a proximal side of the clot, blockage or occlusion. Together the guidewire and microcatheter simultaneously traverse through the clot, blockage or occlusion. Next, the guidewire is retrieved or withdrawn in a proximal direction, replaced by a stentreiver or other mechanical retrieval device advanced through the lumen of the microcatheter. The stentriever or mechanical retrieval device is deployed (enlarged or expanded) typically automatically self-deployed upon exiting from the distal end of the microcatheter. Over time, the clot embeds in the openings of the deployed stentriever, whereupon the microcatheter along with the stentreiver and the clot engaged therein are proximally withdrawn through the lumen of the guide catheter 100. During capture/retrieval and removal of the clot, positioning of the guide catheter within the artery/vessel is maintained by the deployed anchor supporting structure while blood flood is allowed distally beyond the deployed anchor supporting structure. When using the mesh anchor supporting structure of Figures 1-5, its shape (e.g., funnel, cone, cylinder, or combination thereof) provides the added benefit of enclosing the captured clot as it is withdrawn proximally into the guide catheter. In the case of the balloon anchor supporting structure of Figures 6A-6C such benefit is not provided since the arrangement of the inflated (deployed) balloon having a C-shape or U-shape profile partially surrounds the outer perimeter of the guide catheter, thus having on effect on the lumen of the guide catheter through which the captured clot, stentriever and microcatheter are withdrawn during removal.

Numerous benefits are provided by the present inventive anchoring and non-occluding intravascular device over conventional guide catheters that employ a balloon to provide back-up support to the guide catheter. As previously mentioned, an advantage of the present inventive anchoring and non-occluding intravascular device is that there is no occlusion (completely cutting off or entirely stopping) of blood flow distally beyond the deployed anchor supporting structure. Even when deployed the present inventive anchor supporting structure allows the flow of blood distally thereof. Moreover, in those embodiments of the present inventive anchoring and non-occluding intravascular device in which the balloon itself has been eliminated altogether and replaced by a mesh anchor supporting structure, the need for a separate lumen to inflate the balloon is also unnecessary. Elimination altogether of the inflation lumen allows for a larger inner diameter lumen (e.g., approximately 0.082" - approximately 0.091") of the guide catheter able to accommodate ancillary devices (e.g., microcatheters) therein having a larger outer diameter. Because the present inventive anchor supporting structure is deployed before the microcatheter and stentriever are introduced into the lumen of the guide catheter, this novel configuration provides anchoring or restraint of movement of the guide catheter where it really counts (i.e., during clot retrieval and/or aspiration). An additional benefit of the present inventive perforated or mesh anchoring structure is that it may be stiffened by double braiding (e.g., one inner braid forming the lumen and a second outer braid forming a wire protective cage) to prevent or minimize collapse of the distal tip of the guide catheter during aspiration. During retrieval of the microcatheter and stentriever with the clot embedded therein, the funnel (cone) or cylindrical shape of the perforated or mesh structure encases the captured clot, blockage or occlusion thereby improving the performance efficiency of full retrieval.

Yet another benefit of the present inventive anchoring and non-occluding intravascular device over conventional balloon guide catheters is the reduced cost of manufacture in several respects. Balloons of conventional guide catheters are required to remain intact at relative high pressure for a relatively long time and expand with accurate symmetry - all of such manufacturing considerations or requirements of which are not capable of being tested. The present inventive mesh anchor supporting structure is a simple assembly that eliminates the need for such manufacturing requirements. Furthermore, balloons of conventional guide catheters must be manufactured of a relatively soft polymer, exhibiting relative high elongation properties, and a relatively long shelf life to ensure proper expansion. These considerations make the balloon the weak point of the design of conventional balloon guide catheters and are very expensive to manufacture. In contrast, the guide catheter shaft to which the mesh anchor supporting structure is mounted in the present inventive anchor and non-occluding intravascular device has fewer segments than a conventional balloon guide catheter thereby lowering the cost of manufacture.

Since back-up support is provided by the anchor supporting structure rather than the construction of the guide catheter itself, still yet another benefit of the present inventive anchoring and non-occluding intravascular device is that the entire length of the guide catheter may be coated with a hydrophilic coating to assist in trackability.

Thus, while there have been shown, described, and pointed out fundamental novel features of the invention as applied to a preferred embodiment thereof, it will be understood that various omissions, substitutions, and changes in the form and details of the systems/devices illustrated, and in their operation, may be made by those skilled in the art without departing from the spirit and scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

Every issued patent, pending patent application, publication, journal article, book or any other reference cited herein is each incorporated by reference in their entirety.

### ASPECTS OF THE INVENTION

1. A method for using an anchoring and non-occluding intravascular device including a guide catheter including a catheter shaft with a proximal end, an opposite distal end, and a lumen defined longitudinally therein; and an anchor supporting structure deployable from a contracted state to an enlarged state having an enlarged outer diameter relative to that while in the contracted state, wherein while in both the contracted state and the enlarged state the anchor supporting structure is configured to allow passage of blood distally beyond the anchor supporting structure; the method comprising the steps of:
   prior to capture and retrieval of a clot within a vessel, deploying the anchor supporting structure from the contracted state to the enlarged state; while in the enlarged state, the deployed anchor supporting structure anchoring in place the guide catheter in the vessel while still allowing blood flow through the vessel distally beyond the deployed anchor supporting structure; and wherein the anchoring of the guide catheter within the vessel is provided exclusively by the anchor supporting structure.
2. The method of aspect 1, wherein the anchor supporting structure is a mesh anchor supporting structure having a proximal end and an opposite free distal end; the proximal end of the mesh anchor supporting structure is mounted to the distal end of the catheter shaft of the guide catheter.
3. The method of aspect 2, wherein the deploying step comprises using a pushing device advanced through the lumen of the guide catheter to push out from the distal end of the catheter shaft the free distal end of the mesh anchor supporting structure while in the contracted state inverted within itself and received within the lumen at the distal end of the catheter shaft.
4. The method of aspect 2, wherein the mesh anchor supporting structure is cone shape, cylindrical shape, or a combination thereof.
5. The method of aspect 2, wherein the deploying step comprises retracting in a proximal direction a protective sleeve disposed about the catheter shaft of the guide catheter and anchor supporting structure while in the contracted state using a wire.
6. The method of aspect 2, wherein the anchoring and non-occluding intravascular device further comprise a deflated compliant balloon mounted to a distal end of a balloon catheter disposed within the lumen of the guide catheter, wherein the deflated compliant balloon is aligned in a longitudinal direction within the mesh anchor supporting structure; and the deploying step comprises, after the deflated compliant balloon and anchor supporting structure fully emerge from the distal end of the guide catheter, inflating the balloon with inflation media thereby expanding the mesh anchor supporting structure to physically contact and anchor within an inner wall of the vessel the mesh anchor supporting structure and the guide catheter mounted thereto.
7. The method of aspect 1, wherein the anchor supporting structure is a compliant balloon disposed partially about a circumference of the catheter shaft proximate the distal end of the guide catheter; and the deploying step comprises inflating the compliant balloon via inflation media introduced through an inflation lumen defined in the guide catheter; wherein in an inflated state the compliant balloon has a C-shape or U-shape profile including two arm sections each terminating in a free end and an intermediate curved section between the two arm sections.
8. The method of aspect 7, wherein a portion of the intermediate curved section of the inflated compliant balloon physically contacts an inner wall of the vessel anchoring the guide catheter in place within the vessel while spaces defined between the two arm sections of the inflated compliant balloon and the inner wall of the vessel allow blood flow therethrough and distally beyond the inflated compliant balloon.
9. The method of aspect 1, wherein capture and retrieval of the clot comprises aspiration and/or mechanical extraction device.

## Claims

1. An anchoring and non-occluding intravascular device comprising:
a guide catheter including a catheter shaft with a proximal end, an opposite distal end, and a lumen defined longitudinally therein; and
an anchor supporting structure disposed on the guide catheter and deployable from a contracted state to an enlarged state having an enlarged outer diameter relative to that while in a contracted state; wherein in both the contracted and in the enlarged state the anchor supporting structure is configured to allow passage of blood distally beyond the anchor supporting structure; and wherein the anchoring is provided exclusively by the anchor supporting structure.

2. The anchoring and non-occluding intravascular device of claim 1, wherein the anchor supporting structure is a mesh anchor supporting structure having a proximal end and an opposite free distal end, the proximal end of the mesh anchor supporting structure is mounted to the distal end of the catheter shaft of the guide catheter.

3. The anchoring and non-occluding intravascular device of claim 2, wherein, while in the contracted state, the free distal end of the mesh anchor supporting structure is invertible within itself and receivable inside the lumen of the catheter shaft.

4. The anchoring and non-occluding intravascular device of claim 2, wherein the mesh anchor supporting structure is cone shape, cylindrical shape or a combination thereof.

5. The anchoring and non-occluding intravascular device of claim 2, further comprising:
a protective sleeve disposed about the catheter shaft of the guide catheter and anchor supporting structure while in the contracted state; and
a wire secured to a proximal end of the protective sleeve and extending in a proximal direction.

6. The anchoring and non-occluding intravascular device of claim 2, further comprising:
a balloon catheter disposed within the lumen of the guide catheter; wherein the balloon catheter has a catheter shaft with at least one lumen extending axially therethrough for inflating/deflating a compliant balloon mounted to a distal end of the catheter shaft of the balloon catheter and aligned in a longitudinal direction within the anchor supporting structure; wherein the inflated compliant balloon transitioning the anchor supporting structure from the contracted state to the enlarged state.

7. The anchoring and non-occluding vascular device of claim 1, further comprising a compliant balloon disposed proximate the distal end of the catheter shaft of the guide catheter; the compliant balloon being inflatable via an inflation lumen extending longitudinally therein the catheter shaft of the guide catheter; wherein in an inflated state the compliant balloon: (i) has a C-shape or U-shape profile that includes two arm sections each terminating in a free end and an intermediate curved section between the two arm sections; and (ii) is disposed partially around an outer circumference of the catheter shaft of the guide catheter.
